(19) Europäisches Patentamt
European Patent Office
Office européen des brevets

(11) **EP 3 862 040 B1**

## (12) EUROPÄISCHE PATENTSCHRIFT

(45) Veröffentlichungstag und Bekanntmachung des Hinweises auf die Patenterteilung:
**04.09.2024 Patentblatt 2024/36**

(21) Anmeldenummer: **21020042.4**

(22) Anmeldetag: **02.02.2021**

(51) Internationale Patentklassifikation (IPC):
**A61M 16/00** (2006.01)   **A61M 16/10** (2006.01)
**A61B 5/08** (2006.01)   **A61B 5/085** (2006.01)
**A61B 5/083** (2006.01)

(52) Gemeinsame Patentklassifikation (CPC):
(C-Sets verfügbar)
**A61M 16/024; A61B 5/0816; A61B 5/0836;**
**A61B 5/091; A61B 5/14542; A61M 16/1005;**
A61M 2016/1025; A61M 2202/0208; A61M 2205/18;
A61M 2205/3344; A61M 2210/1039;
A61M 2230/205; A61M 2230/432; A61M 2230/435;
A61M 2230/46                           (Forts.)

(54) **BEATMUNGSGERÄT ZUR KÜNSTLICHEN BEATMUNG EINES PATIENTEN**

BREATHING APPARATUS FOR ARTIFICIAL RESPIRATION OF A PATIENT

APPAREIL RESPIRATOIRE DESTINÉ À LA RESPIRATION ARTIFICIELLE D'UN PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.02.2020 DE 102020103094**

(43) Veröffentlichungstag der Anmeldung:
**11.08.2021 Patentblatt 2021/32**

(73) Patentinhaber: **Löwenstein Medical Technology S.A.**
**2557 Luxembourg (LU)**

(72) Erfinder: **Kremeier, Peter**
**76149 Karlsruhe (DE)**

(74) Vertreter: **Marx, Thomas**
**Löwenstein Medical Technology GmbH + Co. KG**
**IP Management**
**Kronsaalsweg 40**
**22525 Hamburg (DE)**

(56) Entgegenhaltungen:
WO-A1-2013/163735    US-A1- 2005 109 340
US-A1- 2007 068 528    US-A1- 2007 163 584
US-A1- 2008 202 525    US-A1- 2012 145 152

• **ABOAB JÉRÔME ET AL: "Effect of inspired oxygen fraction on alveolar derecruitment in acute respiratory distress syndrome", INTENSIVE CARE MEDICINE, SPRINGER BERLIN HEIDELBERG, BERLIN/HEIDELBERG, vol. 32, no. 12, 22 September 2006 (2006-09-22), pages 1979 - 1986, XP037119500, ISSN: 0342-4642, [retrieved on 20060922], DOI: 10.1007/S00134-006-0382-4**

(52) Gemeinsame Patentklassifikation (CPC): (Forts.)

C-Sets
A61M 2230/432, A61M 2230/005;
A61M 2230/435, A61M 2230/005;
A61M 2230/46, A61M 2230/005

**Beschreibung**

Die vorliegende Erfindung betrifft ein Beatmungsgerät zur künstlichen Beatmung eines Patienten.

Bei maschineller Beatmung von Patienten mittels eines Beatmungsgeräts wird dem Patienten Atemgas mit Überdruck zugeführt. Deswegen ist bei der Beatmung der Atemwegsdruck bzw. alveoläre Druck zumindest während der Inspirationsphase größer als der Druck in dem die Lungenbläschen bzw. Alveolen umgebenden Pleuraspalt. Wird in der Exspirationsphase die Druckbeaufschlagung des Atemwegs durch die Beatmungseinrichtung wieder zurückgefahren, so hat dies die Folge, dass sich das Lungengewebe entspannt und der Atemwegsdruck bzw. der alveoläre Druck sinkt. Diese Art der Überdruckbeatmung kann unter bestimmten Umständen dazu führen, dass sich die Druckverhältnisse im Atemweg bzw. in den Alveolen am Ende der Exspirationsphase derart ungünstig einstellen, dass es zu einem Kollaps von Teilen der Alveolen kommt. Dann muss der kollabierte Teil des Lungenvolumens im nachfolgenden Atmungszyklus erst wieder entfaltet werden. Die funktionelle Residualkapazität der Lunge wird dadurch negativ beeinträchtigt, sodass die Sauerstoffsättigung im Blut des Patienten abnimmt. Auch das Lungengewebe kann Schaden nehmen.

So hat jeder Lungenkollaps zur Folge, dass es zwischen den verschlossenen und den offenen Lungengebieten eine instabile Zone gibt, in der die fragilen Lungenbläschen ihren Zustand während eines Atemzugs zyklisch ändern. Sie öffnen sich mit jeder Inspiration und kollabieren, sobald während der Exspiration der Druck in ihnen wieder abnimmt. Dadurch wird die Lunge geschädigt. Diesen Schädigungsmechanismus nennt man zyklisches Rekrutieren (englisch "tidal recruitment"), der in ein sogenanntes Biotrauma, nämlich eine lokale und später systemische Entzündungsreaktion, mündet. Unter einem einfachen Rekrutieren wiederum ist das einfache Öffnen von eventuell kollabierten Teilen bzw. Alveolen der Lunge zu verstehen.

Um einem Kollaps von Alveolen am Ende der Exspirationsphase vorzubeugen, wird bei der maschinellen Überdruckbeatmung regelmäßig ein sogenannter positiver endexspiratorischer Druck (kurz: PEEP) eingestellt.

Bei einer künstlichen Beatmung mit PEEP beaufschlagt das Beatmungsgerät den Atemweg mit einem vorbestimmten Überdruck, dem positiven endexspiratorischen Druck. Der positive endexspiratorische Druck liegt also am Ende der Exspirationsphase noch an.

Dabei ist es wichtig, den positiven endexspiratorischen Druck möglichst so einzustellen, dass während der Exspirationsphase der alveoläre Druck nicht oder jedenfalls nur so weit unterhalb des Drucks im Pleuraspalt liegt, dass das alveoläre Gewebe unter der Wirkung des Drucks im Pleuraspalt nicht kollabiert. Allerdings kann ein zu hoher Wert des eingestellten positiven endexspiratorischen Drucks auch negative Folgen haben, insbesondere während der Inspirationsphase, denn das zu applizierende Atemzugvolumen in der Inspiration baut auf dem PEEP (Restdruck in der Lunge) auf, sodass das Lungengewebe durch das zusätzlich applizierte Lungenvolumen zu sehr hohen Atemwegsdrücken führen kann, was gleichfalls zu einer endinspiratorischen Überdehnung führen kann. Es kommt lokal zu einer zu großen Beanspruchung der Lunge.

Es muss dabei bedacht werden, dass das Lungengewebe sowohl durch einen zu geringen Druck am Ende der Ausatmung als auch durch einen zu hohen Druck am Ende der Einatmung geschädigt wird. Bleiben die Drücke während des gesamten Atemzyklus und über längere Zeit (viele Minuten, Stunden, Tage oder Wochen) zu hoch bzw. zu niedrig, kann sich dies negativ sowohl auf das Herz-Kreislauf-System als auch auf die dünnwandige Gewebestruktur der Lunge auswirken. Vielfältige Komplikationen sind auf Basis dieser mechanischen und biochemischen Auswirkungen denkbar. Das Spektrum reicht von der atemzugsweisen Ausschüttung von Stresshormonen über die Reduzierung der funktionalen Lungenbereiche bis hin zur Zerreißung von Lungengewebe.

Es gilt daher, vor jeder längerdauernden Beatmungstherapie den Spielraum zwischen Überdehnung und Kollaps systematisch auszuloten.

Aus der WO2005092415A1 ist bereits ein Ansatz bekannt, wie auf nicht invasive Weise der Zustand der Lunge beobachtet werden kann, woraus Rückschlüsse für den gewünschten Gasaustausch in der Lunge gezogen werden können.

Die vorliegende Erfindung geht von der WO2005092415A1 aus.

Bei der WO2005092415A1 wird in einem ersten Schritt zunächst versucht, kollabierte Anteile der Lunge mittels einer gezielten Erhöhung der Atemwegsdrücke zu eröffnen, was als Rekrutieren (englisch "recruitment") bezeichnet wird. Diese hohen Drücke bewirken nichts anderes als eine kurzfristige Blähung der Lunge, um möglichst das gesamte Lungengewebe in den Zustand "offen" zu versetzen. Ausgehend hiervon werden die initial hohen Öffnungsdrücke in einem zweiten Schritt rasch und markant auf ein sicheres Niveau abgesenkt, um diese dann in einem dritten Schritt, der sogenannten PEEP-Titration, in kleinen Schritten über einen längeren Zeitraum weiter zu reduzieren.

Während dieses gesamten Vorgangs werden die Lungenmechanik und die Kohlenstoffdioxidkonzentration (kurz: $CO_2$-Konzentration) der ausgeatmeten Luft als patientenspezifische physiologische Parameter gemessen und beobachtet. Die PEEP-Titration gleicht einer technischen Identifikationsroutine und dient dazu, den "Arbeitspunkt" zu ermitteln, also das Druckniveau, auf dem die anschließende Beatmungstherapie über längere Zeit schonend stattfinden kann.

Für die künstliche Beatmung von Patienten ist es also erstrebenswert, die Beanspruchung der Lunge möglichst genau beobachten zu können, um Rückschlüsse auf einen gegebenenfalls zu hohen endinspiratorischen oder zu niedrigen endexspiratorischen Druck oder ganz allgemein auch auf andere, die künstliche Beatmung charakterisierende Parameter zu ziehen. In diesem Sinne werden beim Betreiben eines Beatmungsgeräts zur künstlichen Beatmung eines Patienten also zum einen patientenspezifische physiologische Parameter erfasst und zum anderen technische Beatmungsparameter am Beatmungsgerät festgelegt, auf deren Grundlage das Beatmen des Patienten erfolgt.

US 2012/0145152 A1 offenbart ein medizinisches Gerät zum Überwachen eines Gesundheitszustands eines Patienten, u. a. von dessen Atmung. Dazu kann das Gerät mit einem Kapnographen ausgestattet sein.

US 2008/0202525 A1 offenbart ein Verfahren zum Einstellen einer Atemzuggröße basierend auf einer voreingestellten Ein- und Ausatmungszeit bei einer künstlichen Beatmung.

WO 2013/163735 A1 offenbart eine Vorrichtung und ein Verfahren zum Steuern eines endtidalen Partialdrucks eines Gases in der Lunge einer Person unter Verwendung einer Massenbilanzgleichung.

US 2005/0109340 A1 offenbart eine Vorrichtung und ein Verfahren zum automatischen Steuern eines Beatmungsgeräts, wobei Signale eines Kapnographen als Eingaben verarbeitet werden können.

US 2007/068528 A1 offenbart eine Vorrichtung und ein Verfahren zum Anpassen eines inspiratorischen Drucks und eines positiven endexspiratorischen Drucks unter Berücksichtigung von Änderungen eines alveolären Totraums.

US 2007/163584 A1 offenbart eine Vorrichtung und ein Verfahren zum Steuern eines Beatmungsdrucks abhängig von verschiedenen Lungenpositionen.

Davon ausgehend liegt der vorliegenden Erfindung die Aufgabe zugrunde, ein Beatmungsgerät zur künstlichen Beatmung eines Patienten bereitzustellen, welches auf verlässliche Weise die künstliche Beatmung der Lunge automatisch kontrolliert, sodass weder der Patient unterversorgt ist, noch eine zu große Belastung der Lunge des Patienten durch die künstliche Beatmung auftritt.

Gelöst wird die genannte Aufgabe durch ein Beatmungsgerät gemäß Anspruch 1.

Die Unteransprüche betreffen vorteilhafte Weiterbildungen der vorliegenden Erfindung, deren Merkmale vom Fachmann im Rahmen des technisch Sinnvollen frei miteinander kombiniert werden können.

Im Einzelnen wird ein (nicht beanspruchtes) Verfahren zum Betreiben eines Beatmungsgeräts zur künstlichen Beatmung eines Patienten vorgeschlagen, wobei die folgenden Schritte ablaufen: initiales Erfassen wenigstens eines patientenspezifischen physiologischen Parameters, initiales Festlegen wenigstens eines technischen Beatmungsparameters und Beatmen des Patienten auf Grundlage des technischen Beatmungsparameters. Das Verfahren ist dadurch gekennzeichnet, dass der wenigstens eine technische Beatmungsparameter wenigstens einem der folgenden Beatmungsparameter entspricht: einem Atemminutenvolumen, einem Tidalvolumen, einer Atemfrequenz, einem positiven endexspiratorischen Druck, einer durch das Beatmungsgerät bereitgestellten inspiratorischen Sauerstoffkonzentration. Ferner wird gemäß dem vorschlagsgemäßen Verfahren eine sich wiederholende Messung des wenigstens einen patientenspezifischen physiologischen Parameters vom Beatmungsgerät in Zeitabständen durchgeführt. Schließlich wird weiterhin eine Anpassung des wenigstens einen technischen Beatmungsparameters vom Beatmungsgerät auf Grundlage der sich wiederholenden Messung des patientenspezifischen physiologischen Parameters durchgeführt.

Wesentlich an der vorliegenden Erfindung ist die Erkenntnis, dass der von einem Atemzug zum nächsten Atemzug des künstlich beatmeten Patienten veränderliche Funktionszustand der beatmeten Lungen in die Regelung der Beatmungseinstellungen mit einbezogen wird. Durch die gleichzeitige Einbeziehung sowohl technischer als auch physiologischer Kenngrößen einerseits in Form der technischen Beatmungsparameter und andererseits in Form der patientenspezifischen physiologischen Parameter in die Regelung des Beatmungsgeräts wird eine neue Dimension der Beatmungstherapie erreicht. Dieses Vorgehen ermöglicht eine sichere Steuerung der Beatmungstherapie, selbst unter den verschärften Bedingungen, denen die moderne Intensivmedizin heute ausgesetzt ist, wie abnehmende Liegezeiten der Patienten trotz zunehmend schwerer erkrankter Patienten.

Das entsprechende Beatmungsgerät kann mittels des vorschlagsgemäßen Verfahrens automatisiert den Zustand der Lunge über die patientenspezifischen physiologischen Parameter beobachten und in regelmäßigen Zeitabständen über die vorgeschlagene Regelung automatisch die technischen Beatmungsparameter derart anpassen, dass eine erfolgreiche und auch schonende künstliche Beatmung des Patienten erfolgt.

Nach einer vorteilhaften Ausführungsform des Verfahrens kann der wenigstens eine patientenspezifische physiologische Parameter wenigstens einem der folgenden Parameter entsprechen: einem Atemwegstotraum, einem alveolären Totraum, einem physiologischen Totraum des Patienten.

Nach einer weiteren vorteilhaften Ausführungsform des Verfahrens kann die sich wiederholende Messung des wenigstens einen patientenspezifischen physiologischen Parameters nach jedem Atemzug des Patienten durchgeführt werden. Dadurch kann in besonders vorteilhafter Weise eine Art Echtzeitkontrolle einer sicheren künstlichen Beatmung erfolgen. Die künstliche Beatmung kann über die einzustellenden technischen Beatmungsparameter unmittelbar angepasst werden, sobald die Analyse nach einem Atemzug ergibt, dass der Funktionszustand der Lunge bzw. das Ergebnis der künstlichen Beatmung nicht der bzw. das gewünschte ist.

Dabei ist von besonderem Vorteil, dass vor dem Hintergrund der jeweiligen Beatmungseinstellung für jeden Atemzug der Funktionszustand der Lunge neu ermittelt und die Entscheidung getroffen wird, ob die Lunge bereits optimal schonend beatmet ist oder die Beatmung einer weiteren Optimierung bedarf.

Vorzugsweise kann die sich wiederholende Messung des wenigstens einen patientenspezifischen physiologischen Parameters mittels vorzugsweise volumetrischer Kapnographie durch das Beatmungsgerät erfolgen und der wenigstens eine patientenspezifische physiologische Parameter wenigstens einem unmittelbar den COz-Gasaustausch in der Lunge des Patienten repräsentierenden Parameter, vorzugsweise wenigstens einem der folgenden Parameter entsprechen: einem endexspiratorischen COz-Partialdruck im ausgeatmeten Gasgemisch, einem alveolären COz-Partialdruck, einem bei einem einzelnen Atemzug des Patienten eliminierten Volumen an $CO_2$.

Die Kapnographie ist ein geeignetes Mittel, um die Menge bzw. den Anteil des exspiratorischen Kohlenstoffdioxids, kurz exspiratorisches $CO_2$ genannt, zu bestimmen und auch grafisch darzustellen. Dabei wird die $CO_2$-Kinetik maschinell beatmeter Patienten auf nicht invasive Art und in Echtzeit dargestellt. Insbesondere die volumetrische Kapnographie stellt ein geeignetes Mittel für das klinische Monitoring maschinell beatmeter Patienten dar.

Mittels Kapnographie kann die $CO_2$-Konzentration in Atemgasen während des Atemzyklus gemessen werden. Die $CO_2$-Konzentration wird dabei regelmäßig durch die Absorption von Infrarotlicht gemäß dem Lambert-Beer'schen Gesetz berechnet und üblicherweise als Partialdruck in der Einheit mmHg ausgedrückt. Die grafische Darstellung der COz-Elimination während der Atmung wird als Kapnogramm bezeichnet und das entsprechende Messgerät als Kapnograph.

Sogenannte Nebenstrom-Kapnographen sind Geräte, die eine Atemgasprobe an der Atemwegsöffnung des beatmeten Patienten absaugen und über ein Schlauchsystem zu entfernt von der Ansaugstelle gelegenen Messapparaturen transportieren, um dort das darin enthaltene $CO_2$ zu messen. Sie kommen besser mit feuchten Atemgasen zurecht und verursachen keinen zusätzlichen instrumentellen Totraum. Hauptstrom-Kapnographen hingegen sind Geräte, bei denen sich die COz-Sensoren und in manchen Fällen auch die Flusssensoren in einem Messkopf am Y-Stück des Beatmungsschlauchs des Beatmungsgeräts befinden, sodass mit dieser Methode in situ Messungen nahe der Atemwegsöffnung durchgeführt werden können. Hauptstrom-Kapnographen verursachen keine Volumenverluste und messen das gesamte Atemgasvolumen. Sowohl Nebenstrom-Kapnographen als auch Hauptstrom-Kapnographen können im Rahmen der vorliegenden Erfindung Verwendung finden, wobei die genannten Kapnographen in vorteilhafter Weise bevorzugt einen integralen Bestandteil des entsprechenden Beatmungsgeräts darstellen.

Die Kapnographie wird in der Regel gemäß ihrer grafischen Darstellung klassifiziert, wobei die zeitbasierte oder Standardkapnographie die am meisten verbreitete Art von Kapnogrammen ist. Hierbei wird die $CO_2$-Konzentration im zeitlichen Verlauf geplottet. Die volumenbasierte oder volumetrische Kapnographie hingegen stellt die Menge des bei einem Atemzug eliminierten Kohlendioxids über dem ausgeatmeten Volumen des Atemzugs dar. Im Unterschied zur Standardkapnographie kann die volumetrische Kapnographie in vorteilhafter Weise volumetrische Parameter erfassen, die von klinischer Bedeutung sind. Dazu zählen die pulmonale Elimination von $CO_2$, der Totraum sowie die alveoläre Belüftung. Sowohl die zeitbasierte oder Standardkapnographie als auch die volumenbasierte oder volumetrische Kapnographie können im Rahmen der vorliegenden Erfindung Verwendung finden, wobei die beiden Methoden, um die Elimination von $CO_2$ darzustellen bzw. auszuwerten, auch in Kombination im vorschlagsgemäßen Verfahren bzw. im entsprechenden Beatmungsgerät Anwendung finden können.

Der patientenspezifische physiologische Parameter der Menge des $CO_2$ bzw. konkret des Volumens an $CO_2$, das bei einem einzelnen Atemzug des Patienten eliminiert wird, kann nicht invasiv durch die volumetrische Kapnographie mittels Integration des exspiratorischen $CO_2$ über das exspiratorische Tidalvolumen bestimmt werden.

Im Zuge des vorschlagsgemäßen Verfahrens kann also die Diffusion des $CO_2$ als den Erfolg der künstlichen Beatmung repräsentierender patientenspezifischer physiologischer Parameter herangezogen werden, um automatisiert Rückschlüsse für die Anpassung des wenigstens einen technischen Beatmungsparameters zu ziehen. Eine physiologisch erfolgreiche, aber auch den Patienten schonende künstliche Beatmung kann somit sichergestellt werden.

Nach einer weiteren vorteilhaften Ausführungsform des vorschlagsgemäßen Verfahrens kann die sich wiederholende Messung des wenigstens einen patientenspezifischen physiologischen Parameters mittels vorzugsweise volumetrischer Oxygraphie durch das Beatmungsgerät erfolgen und der wenigstens eine patientenspezifische physiologische Parameter wenigstens einem alveolären $O_2$-Partialdruck und/oder einem Volumen des mit einem Atemzug vom Patienten aufgenommenen Sauerstoffs entsprechen. In analoger Weise zu dem zuvor diskutierten, unmittelbar den $CO_2$-Gasaustausch in der Lunge des Patienten repräsentierenden Parameter als patientenspezifischem physiologischem Parameter können alternativ oder ergänzend in vorteilhafter Weise unmittelbar den Sauerstoffgasaustausch (kurz: $O_2$-Gasaustausch) in der Lunge des Patienten repräsentierende Parameter als patientenspezifische physiologische Parameter eingesetzt werden.

Nach einer weiteren vorteilhaften Ausführungsform des vorschlagsgemäßen Verfahrens kann der wenigstens eine patientenspezifische physiologische Parameter wenigstens einem arteriellen $CO_2$-Partialdruck entsprechen, wobei der arterielle $CO_2$-Partialdruck über nicht invasiv gemessene patientenspezifische physiologische Parameter angenähert wird.

Die Werte des arteriellen $CO_2$-Partialdrucks (kurz: $PaCO_2$) oder aber auch des arteriellen $O_2$-Partialdrucks (kurz: $PaO_2$) sind die Produkte des Gasaustausches in den Lungen und können somit für die Beurteilung der Effizienz des Gasaustausches herangezogen werden. Deren Bestimmung wird klassischerweise jedoch invasiv mittels arterieller Blutgasanalyse (kurz: BGA) vorgenommen. Der normale PaCOz-Wert ist 40 $\pm$ 3 mmHg und Abweichungen von diesem Wert definieren entweder eine Hyperkapnie ($PaCO_2$ > 45 mmHg) oder eine Hypokapnie ($PaCO_2$ < 35 mmHg).

Die wesentlichen Nachteile der Blutgasanalyse sind jedoch, dass sie weder ein nicht invasives noch ein kontinuierliches Messverfahren darstellt. Somit sind ihre Ergebnisse auch nur für den Moment der Blutabnahme repräsentativ. Da die maschinelle Beatmung jedoch eine kontinuierliche Behandlung ist, wären eigentlich nicht invasive, vor allem aber kontinuierliche Überwachungsverfahren zur Beurteilung des Gasaustausches wünschenswert. Hier liefern die Pulsoxymetrie und die Kapnographie orientierende Echtzeitinformationen über die biologisch wichtigsten Gase $O_2$ und $CO_2$, was bei weitgehend gesunden Patienten unter Beatmung zumeist ausreichend ist. Bei komplexeren Pathologien hingegen sollten zu diesen Werten auch noch Blutgasanalysen hinzugezogen werden.

Mittels des vorschlagsgemäßen Verfahrens kann sich auch in nicht invasiver Weise vorteilhaft mittels Kapnographie dem arteriellen $CO_2$-Partialdruck (kurz: $PaCO_2$) weiter genähert werden. Dazu können der arteriell-endtidale $CO_2$-Gradient (kurz: $Pa\text{-}ETCO_2$) oder aber die arteriell-alveoläre $CO_2$-Differenz (kurz: $Pa\text{-}ACO_2$) bestimmt werden. Sodann kann mittels Kapnographie der $PaCO_2$-Wert approximiert werden, indem eine derart berechnete Differenz zu den nicht invasiven $PETCO_2$- oder $PACO_2$-Werten hinzugefügt wird. Die normale $Pa\text{-}ETCO_2$-Differenz liegt bei $\leq$ 5 mmHg und die für $Pa\text{-}ACO_2$ bei ungefähr 5 mmHg bis 8 mmHg. Dabei ist die $Pa\text{-}ACO_2$-Differenz der geeignetere Index, da er den gemittelten Wert des gesamten alveolären Kompartiments repräsentiert, während $PaCO_2$ dies gefäßseitig tut. $Pa\text{-}ACO_2$ ist ähnlich wie der vom Sauerstoff abgeleitete $PA\text{-}aO_2$-Index für die Einschätzung der Diffusion an der alveolokapillären Membran geeignet.

Nach einer weiteren vorteilhaften Ausführungsform des vorschlagsgemäßen Verfahrens kann die sich wiederholende Messung des wenigstens einen patientenspezifischen physiologischen Parameters mittels Pulsoxymetrie erfolgen und der wenigstens eine patientenspezifische physiologische Parameter wenigstens der arteriellen Sauerstoffsättigung des Bluts des Patienten entsprechen.

Nach einer weiteren vorteilhaften Ausführungsform des vorschlagsgemäßen Verfahrens kann als der wenigstens eine patientenspezifische physiologische Parameter der Blutvolumenfluss des intrapulmonalen Rechts-Links-Shunts des Patienten aus einer alveolären Sauerstoffkonzentration, einer Menge des mit einem Atemzug vom Patienten aufgenommenen Sauerstoffs und/oder einer arteriellen Sauerstoffsättigung des Bluts des Patienten bestimmt werden. Der Wert des intrapulmonalen Rechts-Links-Shunts entspricht der Kurzschlussdurchblutung vom sogenannten rechten Herzen zum linken Herzen. Dieses "kurzgeschlossene" Blut fließt von der rechten Herzhälfte zur linken Herzhälfte, ohne am Gasaustausch in der Lunge teilzunehmen. Die Menge, das Volumen oder der Volumenfluss dieses nicht am Gasaustausch teilnehmenden Blutes kann ebenfalls in vorteilhafter Weise dazu dienen, die technischen Beatmungsparameter weiter anzupassen.

Um den Anteil der Kurzschlussdurchblutung in der Lunge (Shunt) zu bestimmen, kann beispielsweise die inspiratorische Sauerstoffkonzentration von hohen Konzentrationen schrittweise auf Raumluftniveau abgesenkt werden und es können die daraus resultierenden korrespondierenden Sauerstoffsättigungswerte ($SpO_2$-Werte) bestimmt werden. Die Wertepaare werden dann miteinander zu einer Kurve verbunden, deren Verlauf mit den auf Basis physiologischer Zusammenhänge zuvor determinierten sogenannten Iso-Shunt-Kurven verglichen wird. Dann wird der dazu passende Shunt-Wert abgelesen. Physiologisch noch aussagekräftigere Shunt-Werte können in einer Weiterbildung des vorschlagsgemäßen Verfahrens erhalten werden, indem in diesen Berechnungen die inspiratorische Sauerstoffkonzentration (kurz: $FiO_2$) durch den tatsächlich gemessenen alveolären Sauerstoffpartialdruck (kurz: $PAO_2$), der wie zuvor beschrieben ermittelt wird, ersetzt wird.

Nach einer weiteren vorteilhaften Ausführungsform des vorschlagsgemäßen Verfahrens kann zu Beginn der künstlichen Beatmung ein Rekrutierungsmanöver durchgeführt werden, wobei über eine initiale Bereitstellung eines erhöhten Beatmungsdrucks eine Lungenüberdehnung erzielt wird und dann der Beatmungsdruck reduziert und eine PEEP-Titration durchgeführt wird. Dadurch kann auf vorteilhafte Weise eine Ablaufsteuerung mit $CO_2$-gestützter Regelung in das Beatmungsgerät integriert werden. Das Rekrutierungsmanöver nebst PEEP-Titration dient dem Zweck, so viel Lungengewebe wie sinnvoll möglich sowohl der atemzugsweisen mechanischen Ventilation als auch dem Gasaustausch zur Verfügung zu stellen. Durch dieses systematische Vorgehen werden Zustände der Lungenüberdehnung wie auch des Lungenkollapses identifiziert und können anschließend gezielt durch Anpassungen an den Beatmungseinstellungen vermieden werden.

Nach einer weiteren vorteilhaften Ausführungsform des vorschlagsgemäßen Verfahrens kann während der künstlichen Beatmung die Beatmungsdruckamplitude permanent überwacht werden, wobei ein Alarm ausgelöst wird, wenn eine voreingestellte maximale Beatmungsdruckamplitude überschritten wird. Über eine solche Überwachung der Beatmungsdruckamplitude mit entsprechenden Alarmfunktionen können sowohl frühzeitig Hinweise auf den Verlust funktionellen Lungenvolumens gegeben werden als auch so früh wie möglich geeignete Therapiemaßnahmen ergriffen werden. Diese beinhalten nicht nur solche, die mittels des Beatmungsgeräts direkt durchgeführt werden können, sondern auch solche, die der Intervention seitens des klinischen Personals bedürfen. Diese Maßnahmen können umfassen: weitergehende diagnostische Maßnahmen (z. B. Lungenultraschall, CT-Untersuchung), gezielte Patientenlagerung, Drainage von freier Luft oder Flüssigkeiten.

Nach einem weiteren Aspekt der Lehre wird ein Beatmungsgerät zur künstlichen Beatmung eines Patienten gemäß Anspruch 1 vorgeschlagen. Das vorschlagsgemäße Beatmungsgerät umfasst: eine Messeinrichtung, die zum Erfassen wenigstens eines patientenspezifischen physiologischen Parameters eingerichtet ist; eine Steuereinrichtung, die zum Festlegen wenigstens eines technischen Beatmungsparameters eingerichtet ist, wobei auf Grundlage des technischen Beatmungsparameters das Beatmen des Patienten erfolgt, wobei der wenigstens eine technische Beatmungsparameter wenigstens einem der folgenden Beatmungsparameter entspricht: einem Atemminutenvolumen, einem Tidalvolumen, einer Atemfrequenz, einem positiven endexspiratorischen Druck, einer durch das Beatmungsgerät bereitgestellten inspiratorischen Sauerstoffkonzentration; sowie eine Regelungseinheit, die in Kommunikation mit der Messeinrichtung und mit der Steuereinrichtung ist. Die Messeinrichtung ist dabei derart eingerichtet, dass sie eine sich wiederholende Messung des wenigstens einen patientenspezifischen physiologischen Parameters in Zeitabständen durchführt. Die Regelungseinheit ist derart eingerichtet, dass sie eine Anpassung des wenigstens einen technischen Beatmungsparameters auf Grundlage der sich wiederholenden Messung des patientenspezifischen physiologischen Parameters durchführt.

Das vorschlagsgemäße Beatmungsgerät zur künstlichen Beatmung eines Patienten ist vorzugsweise dazu eingerichtet, mittels eines vorbeschriebenen Verfahrens zum Betreiben eines Beatmungsgeräts betrieben zu werden.

Die konkreten Auswirkungen und Vorteile dieses Verfahrens sind zuvor bereits hinsichtlich des Verfahrens zum Betreiben eines Beatmungsgeräts beschrieben worden. Insofern wird auf diese Bezug genommen.

Insbesondere ist im Hinblick auf das Beatmungsgerät die Erkenntnis wesentlich, dass der von einem Atemzug zum nächsten Atemzug des künstlich beatmeten Patienten veränderliche Funktionszustand der beatmeten Lungen in die Regelung der Beatmungseinstellungen mit einbezogen wird. Durch die gleichzeitige Einbeziehung sowohl technischer als auch physiologischer Kenngrößen einerseits in Form der technischen Beatmungsparameter und andererseits in Form der patientenspezifischen physiologischen Parameter in die Regelung des Beatmungsgeräts wird eine neue Dimension der Beatmungstherapie erreicht. Dieses Vorgehen ermöglicht eine sichere Steuerung der Beatmungstherapie, selbst unter den verschärften Bedingungen, denen die moderne Intensivmedizin heute ausgesetzt ist, wie abnehmende Liegezeiten der Patienten trotz zunehmend schwerer erkrankter Patienten.

Das vorschlagsgemäße Beatmungsgerät kann automatisiert den Zustand der Lunge über die patientenspezifischen physiologischen Parameter beobachten und in regelmäßigen Zeitabständen über die vorgeschlagene Regelung automatisch die technischen Beatmungsparameter derart anpassen, dass eine erfolgreiche und auch schonende künstliche Beatmung des Patienten erfolgt.

Nach einer vorteilhaften Ausführungsform des Beatmungsgeräts ist vorgesehen, dass der wenigstens eine patientenspezifische physiologische Parameter wenigstens einem der folgenden Parameter entspricht: einem Atemwegstotraum, einem alveolären Totraum, einem physiologischen Totraum des Patienten.

Nach einer weiteren vorteilhaften Ausführungsform des Beatmungsgeräts ist vorgesehen, dass die Messeinrichtung ferner derart eingerichtet ist, dass sie die sich wiederholende Messung des wenigstens einen patientenspezifischen physiologischen Parameters nach jedem Atemzug des Patienten durchführt.

Nach einer weiteren vorteilhaften Ausführungsform des Beatmungsgeräts ist vorgesehen, dass die Messeinrichtung als Kapnograph ausgebildet ist und derart eingerichtet ist, dass sie die sich wiederholende Messung des wenigstens einen patientenspezifischen physiologischen Parameters mittels volumetrischer Kapnographie durchführt, und dass der wenigstens eine patientenspezifische physiologische Parameter wenigstens einem unmittelbar den $CO_2$-Gasaustausch in der Lunge des Patienten repräsentierenden Parameter, vorzugsweise wenigstens einem der folgenden Parameter entspricht: einem endexspiratorischen $CO_2$-Partialdruck im ausgeatmeten Gasgemisch, einem alveolären $CO_2$-Partialdruck, einem bei einem einzelnen Atemzug des Patienten eliminierten Volumen an $CO_2$.

Nach einer weiteren vorteilhaften Ausführungsform des Beatmungsgeräts ist vorgesehen, dass die Messeinrichtung derart eingerichtet ist, dass sie die sich wiederholende Messung des wenigstens einen patientenspezifischen physiologischen Parameters mittels vorzugsweise volumetrischer Oxygraphie durchführt, und dass der wenigstens eine patientenspezifische physiologische Parameter wenigstens einem alveolären $O_2$-Partialdruck und/oder einem Volumen des mit einem Atemzug vom Patienten aufgenommenen Sauerstoffs entspricht.

Nach einer weiteren vorteilhaften Ausführungsform des Beatmungsgeräts ist vorgesehen, dass der wenigstens eine patientenspezifische physiologische Parameter wenigstens dem arteriellen $CO_2$-Partialdruck entspricht, wobei die Messeinrichtung derart eingerichtet ist, dass sie den arteriellen $CO_2$-Partialdruck über nicht invasiv gemessene patientenspezifische physiologische Parameter annähert.

Nach einer weiteren vorteilhaften Ausführungsform des Beatmungsgeräts ist vorgesehen, dass die Messeinrichtung als Pulsoxymeter ausgebildet ist und derart eingerichtet ist, dass sie die sich wiederholende Messung des wenigstens einen patientenspezifischen physiologischen Parameters mittels Pulsoxymetrie durchführt, und dass der wenigstens eine patientenspezifische physiologische Parameter wenigstens der arteriellen Sauerstoffsättigung des Bluts des Patienten entspricht.

Nach einer weiteren vorteilhaften Ausführungsform des Beatmungsgeräts ist vorgesehen, dass die Messeinrichtung derart eingerichtet ist, dass sie als den wenigstens einen patientenspezifischen physiologischen Parameter den Blutvolumenfluss des intrapulmonalen Rechts-Links-Shunts des Patienten aus einer alveolären Sauerstoffkonzentration, einer Menge des mit einem Atemzug vom Patienten aufgenommenen Sauerstoffs und/oder einer arteriellen Sauerstoffsättigung des Bluts des Patienten bestimmt.

Nach einer weiteren vorteilhaften Ausführungsform des Beatmungsgeräts ist vorgesehen, dass die Steuereinrichtung derart eingerichtet ist, dass sie zu Beginn der künstlichen Beatmung ein Rekrutierungsmanöver durchführt, wobei über eine initiale Bereitstellung eines erhöhten Beatmungsdrucks eine Lungenüberdehnung erzielt wird und dann der Beatmungsdruck reduziert und eine PEEP-Titration durchgeführt wird.

Nach einer weiteren vorteilhaften Ausführungsform des Beatmungsgeräts ist vorgesehen, dass eine Überwachungseinheit vorgesehen ist, die derart eingerichtet ist, dass die Beatmungsdruckamplitude während der künstlichen Beatmung permanent überwacht wird, wobei ein Alarm ausgelöst wird, wenn eine voreingestellte maximale Beatmungsdruckamplitude überschritten wird.

Die Vorteile des vorschlagsgemäßen Beatmungsgeräts bzw. seiner bevorzugten Ausführungsformen werden mit Blick auf das zuvor beschriebene vorschlagsgemäße Verfahren zum Betreiben eines Beatmungsgeräts deutlich, sodass insofern auf die Beschreibung des Verfahrens Bezug genommen wird.

Bevorzugte Ausführungsformen der Erfindung werden beispielhaft anhand der beiliegenden Figuren beschrieben. Es zeigt

Figur 1    ein Beispieldiagramm, anhand dessen der Rechts-Links-Shunt bei einem Patienten ermittelt werden kann;

Figur 2    ein typisches Diagramm der volumetrischen Kapnographie und die wichtigsten daraus ableitbaren Werte für die künstliche Beatmung;

Figur 3    zwei Diagramme, die die zeitbasierte Kapnographie (links) und die volumenbasierte Kapnographie (rechts) darstellen;

Figur 4    zwei Diagramme, die die zeitbasierte Kapnographie (links) und die volumenbasierte Kapnographie (rechts) mit den Totraumanteilen darstellen;

Figur 5    ein Monitoring der alveolären Ventilation anhand der volumetrischen Kapnographie.

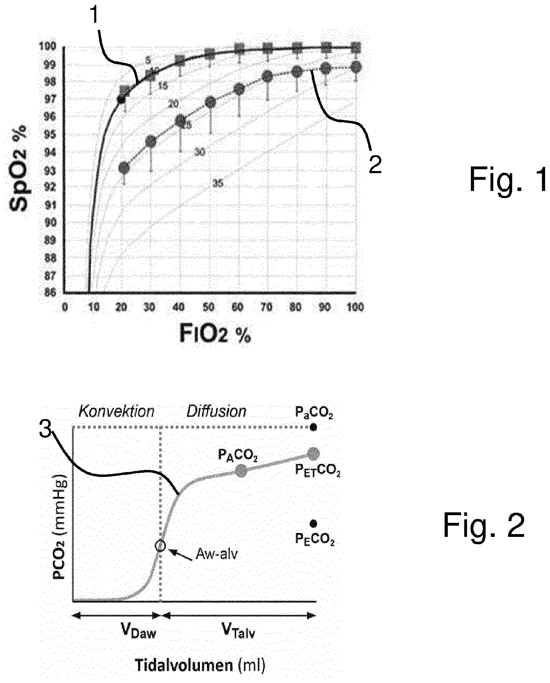

In Figur 1 sind zwei beispielhafte Kurven 1, 2 dargestellt, wobei die Wertepaare einmal mit Quadraten bei der Kurve 1 und einmal mit Kreisen bei der Kurve 2 gekennzeichnet wurden. Dabei sind gemessene Sauerstoffsättigungswerte (kurz: $SpO_2$-Werte) eines künstlich beatmeten Patienten auf der y-Achse bei verschiedenen inspiratorischen Sauerstoffkonzentrationen (kurz: $FiO_2$), welche durch das Beatmungsgerät bereitgestellt wurden, auf der x-Achse eingetragen.

Um den Anteil der Kurzschlussdurchblutung in der Lunge, den sogenannten Rechts-Links-Shunt, zu bestimmen, wird die inspiratorische Sauerstoffkonzentration $FiOz$ in einem Beatmungsvorgang von hohen Konzentrationen schrittweise auf Raumluftniveau abgesenkt und es werden die daraus resultierenden korrespondierenden $SpO_2$-Werte über die Messeinrichtung des vorschlagsgemäßen Beatmungsgeräts bestimmt. Diese Punkte werden dann miteinander zu einer Kurve 1 bzw. 2 verbunden, deren Verlauf mit den auf Basis physiologischer Zusammenhänge zuvor determinierten Iso-Shunt-Kurven verglichen wird. Dann wird der dazu passende Shunt-Wert abgelesen.

Diese Iso-Shunt-Kurven sind als feine Linien mit den dazugehörigen Shunt-Werten im Diagramm in Figur 1 eingetragen. Aus der Übereinstimmung der gemessenen Kurven mit den Iso-Shunt-Kurven können die genäherten Shunt-Werte abgelesen werden. Vorliegend entspricht die Kurve 1 einem Rechts-Links-Shunt von ca. 24 %, während die Kurve 1 einem Rechts-Links-Shunt von unter 10 % entspricht. Figur 2 zeigt ein Diagramm der volumenbasierten Kapnographie und deren wichtigste Parameter. Ein solches Diagramm kann integriert in dem vorschlagsgemä-ßen Beatmungsgerät erstellt werden und darüber hinaus über eine Anzeigeeinheit, vorzugsweise ein Display, dem behandelnden Medizinpersonal angezeigt werden.

In dem Diagramm ist auf der y-Achse der gemessene $CO_2$-Partialdruck $PCO_2$ der ausgeatmeten Luft in der Einheit mmHg und auf der x-Achse das Tidalvolumen $V_T$ in der Einheit ml aufgetragen. Anhand der aufgezeichneten Kurve 3 können verschiedene - für die künstliche Beatmung wichtige - patientenspezifische physiologische Parameter abgelesen werden.

Beispiele dafür sind die folgenden klinisch wichtigen $CO_2$-Partialdrücke: $PETCO_2$ als endtidaler $CO_2$-Partialdruck, $PACO_2$ als mittlerer alveolärer $CO_2$-Partialdruck und $P\overline{E}CO_2$ als der gemischt exspiratorische $CO_2$-Partialdruck. Der gemischt exspiratorische $CO_2$-Partialdruck $P\overline{E}CO_2$ ergibt sich aus dem Verdünnungseffekt, der durch den physiologischen Totraum $V_{Dphys}$ entsteht. Dies sind alles patientenspezifische physiologische Parameter, die regelmäßig durch die Messeinrichtung des Beatmungsgeräts gemessen werden können und dann als Grundlage für die Anpassung der technischen Beatmungsparameter dienen können.

Weitere patientenspezifische physiologische Parameter sind $PaCO_2$ als der $CO_2$-Partialdruck im arteriellen Blut, der mittels Blutprobe analysiert wird und als gepunktete Linie oben im Kapnogramm erscheint. Der mathematische Wende- oder Umschlagspunkt des Kapnogramms - die Schnittstelle zwischen Atemwegen und Alveolen - ist die Grenze zwischen dem Atemwegstotraum $V_{Daw}$ und dem alveolären Tidalvolumen $V_{Talv}$.

Das Totraumvolumen ist im Allgemeinen darauf zurückzuführen, dass bei jedem Atemzyklus ein Anteil des Tidalvolumens $V_T$ in den luftleitenden Atemwegen verbleibt und so das alveoläre Kompartiment nicht erreicht. Dieser Anteil wird Atemwegstotraum $V_{Daw}$ genannt. Bei künstlich beatmeten Patienten wird häufig ein zusätzlicher instrumenteller Totraum $V_{Dinst}$ vorgefunden, der durch Komponenten des Beatmungsgeräts wie Konnektoren, Winkelstücke oder Befeuchtungs- oder Keimfilter verursacht wird, die zwischen dem Y-Stück und dem Endotrachealtubus bei dem Beatmungsgerät platziert sind. Hinzu kommt noch der sogenannte alveoläre Totraum $V_{Dalv}$. Dieser resultiert aus Alveolen, die zwar belüftet, aber nicht perfundiert werden. Diese Alveolen nehmen demnach auch nicht am Gasaustausch teil. Der Atemwegstotraum $V_{Daw}$ und der alveoläre Totraum $V_{Dalv}$ bestimmen zusammen den globalen Totraum $V_D$, der auch als physiologischer Totraum $V_{Dphys}$ bezeichnet wird.

Der globale Totraum $V_D$ kann mittels volumetrischer Kapnographie bestimmt werden. Hierzu wird das sogenannte Fowler-Konzept, basierend auf der Bohr-Formel, herangezogen. Fowler postulierte, dass die Grenze zwischen dem konvektiven Gastransport in den luftleitenden Atemwegen (Totraum) und dem diffusiven Gastransport im alveolären Kompartiment durch die Schnittstelle zwischen Atemwegen und Alveolen gebildet wird, welche wiederum durch den Wendepunkt des $CO_2$-Verlaufs im Kapnogramm repräsentiert wird (vgl. Figur 2). Dieser Ansatz vermag es, den Atemwegstotraum $V_{Daw}$ eines jeden Atemzugs repetitiv auf nicht invasive Weise zu ermitteln.

Die Formel nach Bohr ist eine Massenbilanzgleichung, die den Anteil des Tidalvolumens $V_T$, der kein $CO_2$ enthält, wie folgt berechnet:

$$V_{Dphys}/V_T = V_D/V_T = (PACO_2 - P\bar{E}CO_2) / (PACO_2 - PICO_2)$$

wobei $PICO_2$ der inspirierte $CO_2$-Partialdruck ist, bei dem unter normalen Umständen von einem Wert von null ausgegangen wird, weil das Frischgas kein $CO_2$ enthalten sollte. Für eine adäquate Totraumberechnung muss dieser Wert jedoch immer mitberücksichtigt werden, weil ein Teil der $CO_2$-Rückatmung aus dem Y-Stück des Beatmungsgeräts und einem zusätzlichen instrumentellen Totraum $V_{Dinst}$ stammt. Schließlich wird der alveoläre Totraum $V_{Dalv}$ durch Subtraktion des Atemwegstotraums $V_{Daw}$ vom physiologischen Totraum $V_{Dphys}$ berechnet.

Der Totraum $V_D$ bzw. $V_{Dphys}$ kann als Teil der Minutenventilation in l/min oder als absoluter Wert eines Atemzugs, als physiologischer Totraum $V_{Dphys}$, in der Einheit ml oder aber als Verhältnis zum Atemzugvolumen $V_D/V_T$ ausgedrückt werden. Am besten geeignet ist letzteres Verhältnis, da der Totraum $V_D$ bzw. $V_{Dphys}$ durch die Größe des Tidalvolumens $V_T$ stark beeinflusst wird. Die auf das exspirierte Volumen normalisierten Totraumverhältnisse ermöglichen den Vergleich zwischen Personen unterschiedlicher Körpermasse, die mit unterschiedlichen Tidalvolumina $V_T$ beatmet werden.

Für die klinische Anwendungen ist es wichtig, die physiologischen Totraumwerte $V_{Dphys}$ zu kennen. Bei gesunden und jungen, spontan atmenden Menschen beträgt das Verhältnis physiologischer Totraum $V_{Dphys}$ zu Tidalvolumen $V_T$ (kurz: $V_{Dphys}/V_T$) etwa 20-25 %, aufgeteilt in die Verhältnisse Atemwegstotraum $V_{Daw}$ zu Tidalvolumen $V_T$ (kurz: $V_{Daw}/V_T$) mit ca. 15-20 % und alveolärer Totraum $V_{Dalv}$ zu Tidalvolumen $V_T$ (kurz: $V_{Dalv}/V_T$) mit rund 5-9 %.

Bei lungengesunden Patienten steigt durch die maschinelle Beatmung das Verhältnis physiologischer Totraum $V_{Dphys}$ zu Tidalvolumen $V_T$ (kurz: $V_{Dphys}/V_T$) auf 30-40 % an, während dieser Wert bei Intensivpatienten typischerweise bei über 40 % liegt. Bei Patienten mit chronischen Lungenerkrankungen zeigen sich erhöhte physiologische Toträume $V_{Dphys}$ von mehr als 50 %, wohingegen der Atemwegstotraum $V_{Daw}$ und der alveoläre Totraum $V_{Dalv}$ gegenüber dem Normalwert um das Zwei- bis Dreifache erhöht sein können.

In einem Beitrag von Enghoff (Enghoff H. Volum inefficax. Bemerkungen zur Frage des schädlichen Raumes. Upsala Läk Fören Förch 1938; 44: 191-218) wurde die Bohr'sche Formel modifiziert, indem der alveoläre $COz$-Partialdruck $PACO_2$ durch den arteriellen $COz$-Partialdruck $PaCOz$ ersetzt wurde, weil der alveoläre $CO_2$-Partialdruck $PACO_2$ bettseitig nicht messbar war. Diese invasive und intermittierende Berechnungsmethode unter Hinzunahme der Blutgasanalyse überschätzt jedoch den Totraum $V_D$ systematisch, weil der Wert des arteriellen $CO_2$-Partialdrucks $PaCO_2$ normalerweise immer höher ausfällt als der durch den Totraum $V_D$ und den Shunt-Effekt beeinflusste alveoläre $CO_2$-Partialdruck $PACO_2$. Durch das vorschlagsgemäße Verfahren kann die Formel nach Bohr jedoch vollständig nicht invasiv in der Praxis angewendet werden, denn die Bestimmung des alveolären $CO_2$-Partialdrucks $PACO_2$ und des gemischt exspiratorischen $CO_2$-Partialdrucks $P\bar{E}CO_2$ ist aus dem volumetrischen Kapnogramm technisch möglich und wird integriert in dem vorschlagsgemäßen Beatmungsgerät durchgeführt.

Mit dem hier dargestellten Werkzeug der volumetrischen Kapnographie kann heute der Totraumanteil am Atemzugvolumen und somit die Effizienz der Ventilation bettseitig ermittelt werden, wobei eine zusätzliche arterielle Blutgasanalyse eine zusätzliche verlässliche Abschätzung des Shunts liefern kann.

Die Kapnographie liefert anhand ihrer spannungsbasierten Werte gänzlich nicht invasiv Informationen über die $CO_2$-Diffusion. Der endexspiratorische $CO_2$-Partialdruck PETCO$_2$ im ausgeatmeten Gasgemisch ist der bekannteste Parameter der Kapnographie. Er repräsentiert den $CO_2$-Partialdruck in peripheren Lungenabschnitten bzw. in solchen mit langer Zeitkonstante, deren Leerung in die großen Atemwege verspätet erfolgt. Im Gegensatz dazu repräsentiert der alveoläre $CO_2$-Partialdruck PACO$_2$ alle Lungeneinheiten mit unterschiedlichen Zeitkonstanten. Über viele Jahre wurde angenommen, dass dieser Wert nicht bettseitig gemessen werden könne. Mittels des vorschlagsgemäßen Verfahrens kann der alveoläre $CO_2$-Partialdruck PACO$_2$ als Mittelpunkt der Steigung von der letzten Phase der in Figur 2 dargestellten Kurve des Kapnogramms genau bestimmt werden. Der Grund hierfür ist, dass ein solcher Punkt den gemittelten $CO_2$-Wert in allen Alveolen darstellen muss, weil diese letzte Phase nur durch die Gase in den Alveolen determiniert wird. Auf diese Weise können vorteilhaft mittels des vorschlagsgemäßen Verfahrens die alveoläre Ventilation und die Toträume atemzugsweise und nicht invasiv bestimmt werden.

Der alveoläre $CO_2$-Partialdruck PACO$_2$ kann nun, neben dem endexspiratorischen $CO_2$-Partialdruck PETCO$_2$ im ausgeatmeten Gasgemisch, ebenfalls zur Approximierung des arteriellen $CO_2$-Partialdrucks PaCO$_2$ herangezogen werden. Es muss jedoch immer klar sein, dass im Endeffekt der klinisch bedeutende arterielle $CO_2$-Partialdruck PaCO$_2$ weder durch den endexspiratorischen $CO_2$-Partialdruck PETCO$_2$ im ausgeatmeten Gasgemisch noch durch den alveolären $CO_2$-Partialdruck PACO$_2$ ersetzt werden kann. Grund hierfür sind sowohl Shunt- also auch Totraumeffekte. Beide führen dazu, dass der arterielle $CO_2$-Partialdruck PaCO$_2$ gewöhnlich höher ist als der alveoläre $CO_2$-Partialdruck PACO$_2$ und der endexspiratorische $CO_2$-Partialdruck PETCO$_2$ im ausgeatmeten Gasgemisch. Dies gilt wegen des Vorliegens eines anatomischen Shunts und Totraums sogar für junge Menschen mit gesunden Lungen. Daher sollte in kritischen Situationen, und insbesondere dann, wenn wesentliche Änderungen der $CO_2$-Kinetik bemerkt oder vermutet werden, ergänzend eine Analyse der Blutgase durchgeführt werden.

Gemäß dem vorschlagsgemäßen Verfahren kann sich jedoch mittels Kapnographie dem arteriellen $CO_2$-Partialdruck PaCO$_2$ weiter angenähert werden. Dies kann erzielt werden, indem der arteriell-endtidale $CO_2$-Gradient Pa-ETCO$_2$ oder aber die arteriell-alveoläre $CO_2$-Differenz Pa-ACO$_2$ bestimmt wird. Man kann mittels Kapnographie den arteriellen $CO_2$-Partialdruck PaCO$_2$ approximieren, indem eine derart berechnete Differenz zu den nicht invasiven Werten des endexspiratorischen $CO_2$-Partialdrucks PETCO$_2$ im ausgeatmeten Gasgemisch oder des alveolären $CO_2$-Partialdrucks PACO$_2$ hinzugefügt wird.

Der normale Differenzwert für den arteriell-endtidalen $CO_2$-Gradienten Pa-ETCO$_2$ liegt bei $\leq 5$ mmHg und der normale Wert für die arteriell-alveoläre $CO_2$-Differenz Pa-ACO$_2$ liegt bei ca. 5-8 mmHg. Dabei ist die arteriell-alveoläre $CO_2$-Differenz Pa-ACO$_2$ der geeignetere Index, da er den gemittelten Wert des gesamten alveolären Kompartiments repräsentiert, während der arterielle $CO_2$-Partialdruck PaCO$_2$ dies gefäßseitig tut. Die arteriell-alveoläre $CO_2$-Differenz Pa-ACO$_2$ ist somit für die Einschätzung der Diffusion an der alveolokapillären Membran geeignet.

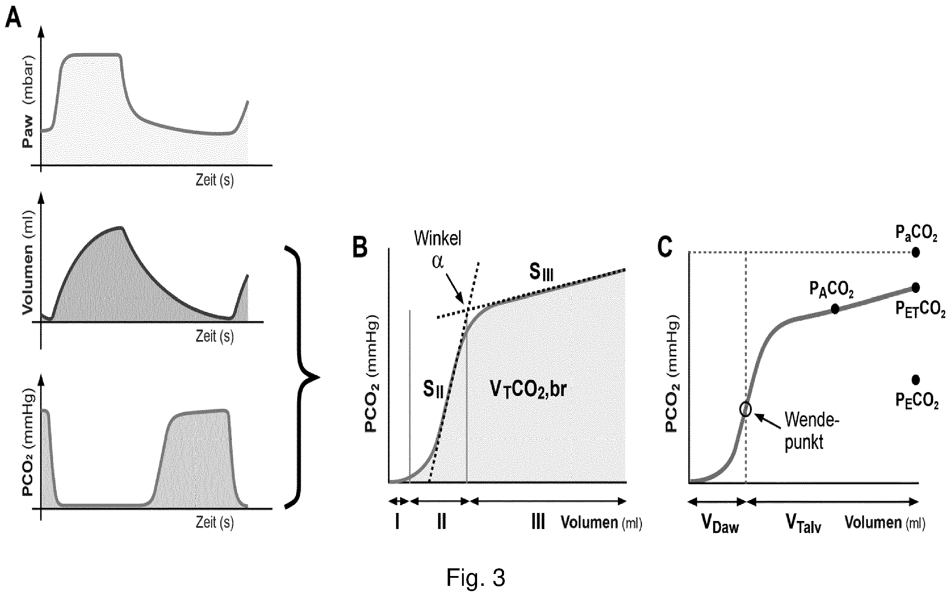

In Figur 3 sind in der linken Hälfte die zeitbasierte Kapnographie und in der rechten Hälfte die volumenbasierte Kapnographie gegenübergestellt. Das volumetrische Kapnogramm rechts lässt sich, analog zum Standardkapnogramm, in die folgenden drei Phasen unterteilen: Phase I stellt den ersten Teil der Exspiration dar, in dem kein $CO_2$ enthalten ist. In dieser Beatmungsphase I werden ungefähr 10-12 % des Tidalvolumens $V_T$ bereitgestellt. Phase II zeigt einen raschen $CO_2$-Anstieg während der Exspiration. In dieser Beatmungsphase II werden ungefähr die nächsten 15-18 % des Tidalvolumens $V_T$ bereitgestellt. Schließlich wird die Phase III erreicht, in welcher der Anteil des Tidalvolumens $V_T$, der nur durch die Gase, die aus den Alveolen kommen, bestimmt wird, die verbleibenden 70-75 % des Tidalvolumens $V_T$ ausmacht.

Die volumetrische Kapnographie bzw. das Kapnogramm kann durch die Steigungen zweier sich kreuzender Geraden näher charakterisiert werden. Die eine Gerade ist die Steigung von Phase II und wird durch den Kennwert $S_{II}$ charakterisiert. Der Normalwert von $S_{II}$ beträgt 0,36-0,40 mmHg/ml und wird durch die verschiedenen Raten der Entleerung von $CO_2$ aus unterschiedlichen Lungeneinheiten in die großen Atemwege bestimmt. Die andere Gerade ist die Steigung von Phase III und wird durch den Kennwert $S_{III}$ charakterisiert. Der Normalwert von $S_{III}$ beträgt 0,007-0,017 mmHg/ml, wobei $S_{III}$ hauptsächlich von der Verteilung von Belüftung und Perfusion in den Lungen bestimmt wird. Der Alpha-Winkel ist der Winkel, der an der Schnittstelle der Geraden $S_{II}$ und $S_{III}$ gebildet wird, wobei der Normalwert für den Alpha-Winkel 150-160° beträgt.

Die Fläche unter der Kurve ist der wichtigste Parameter. Die Fläche stellt die $CO_2$-Menge dar, die in einem Atemzug eliminiert wird, und wird mit dem Zeichen VTCO$_{2,br}$ gekennzeichnet. Dieser Wert hängt vor allem von patientenspezifischen Faktoren und der Größe des Tidalvolumens $V_T$ ab. Ein typischer Wert des Tidalvolumens $V_T$ beträgt bei einem Erwachsenen ca. 10-30 ml.

Weiter unterscheidet man partialdruckbasierte Werte. Diese sind wichtige Kenngrößen des exspiratorischen $CO_2$, die für die Berechnung klinischer Variablen wie z. B. des Totraums $V_D$ verwendet werden. Der endexspiratorische

$CO_2$-Partialdruck $PETCO_2$ im ausgeatmeten Gasgemisch hat Normalwerte, die im vorliegend dargestellten Beispiel bei ca. 33-37 mmHg liegen. $PACO_2$ ist der mittlere alveoläre $CO_2$-Partialdruck, der aufgrund der positiven Steigung in Phase III mit Werten von ca. 30-35 mmHg in der Regel niedriger ist als der entsprechende endexspiratorische $CO_2$-Partialdruck $PETCO_2$ im ausgeatmeten Gasgemisch. $P\bar{E}CO_2$ ist der gemischt exspiratorische $CO_2$-Partialdruck, der sich aus dem Verdünnungseffekt ergibt, der durch den physiologischen Totraum $V_{Dphys}$ entsteht, wobei der gemischt exspiratorische $CO_2$-Partialdruck $P\bar{E}CO_2$ Werte von ca. 18-24 mmHg annimmt.

Der mathematische Wendepunkt (d. h. der Punkt, an dem sich das Krümmungsverhalten der Kapnogrammkurve ändert) stellt den Mittelwert der stationären Schnittstellen zwischen dem konvektiven und dem diffusiven $CO_2$-Transport in den Lungen dar. Am Ende der Inspiration befindet sich dieser anatomisch in der Nähe der respiratorischen Bronchiolen. Diese Schnittstelle zwischen Atemwegen und Alveolen bestimmt den Übergang zwischen den Atemwegen und den alveolären Kompartimenten.

Sowohl die zeitbasierte als auch die zeit- und volumenbasierte Kapnographie liefert somit mit jedem Atemzug bettseitig und nicht invasiv komplementäre Informationen über alle wichtigen Körperfunktionen, die an der $CO_2$-Kinetik beteiligt sind.

Durch das vorschlagsgemäße Verfahren sowie das vorschlagsgemäße Beatmungsgerät werden nun Hilfsmittel für die künstliche Beatmung bereitgestellt, mittels derer die künstliche Beatmung, insbesondere durch die Zuhilfenahme der Messmethoden der Kapnographie, sicherer ablaufen kann. Die Kapnographie bietet dabei ein vorteilhaftes Monitoring für maschinell beatmete Patienten. Die zeitbasierte Kapnographie beschreibt das exspiratorische $CO_2$ in seiner zeitlichen Abfolge und liefert dabei Echtzeitinformationen über die verabreichten Atemhübe sowie qualitative Informationen zur $CO_2$-Kinetik des Organismus. Die volumenbasierte Kapnographie liefert dagegen klinisch relevante volumetrische Daten zur $CO_2$-Kinetik. Beide Methoden der Kapnographie sind bettseitig verfügbar, nicht invasiv und liefern komplementäre, klinisch relevante Informationen. Die volumenbasierte Kapnographie stellt einen wesentlichen Fortschritt gegenüber der heute standardmäßigen Messung der Atemmechanik hinaus, weil sie in der Lage ist, Kenngrößen des Gasaustausches zu liefern. Die volumenbasierte Kapnographie misst nicht nur die durch die Ventilation zu eliminierende $CO_2$-Last, sondern beschreibt sowohl die Effizienz als auch die hämodynamischen Auswirkungen jedes beliebigen Beatmungsmodus und der entsprechenden Beatmungseinstellungen. Die volumenbasierte Kapnographie, integriert in das vorschlagsgemäße Beatmungsgerät, eröffnet somit eine neue Dimension des Monitorings der Beatmung und der Hämodynamik.

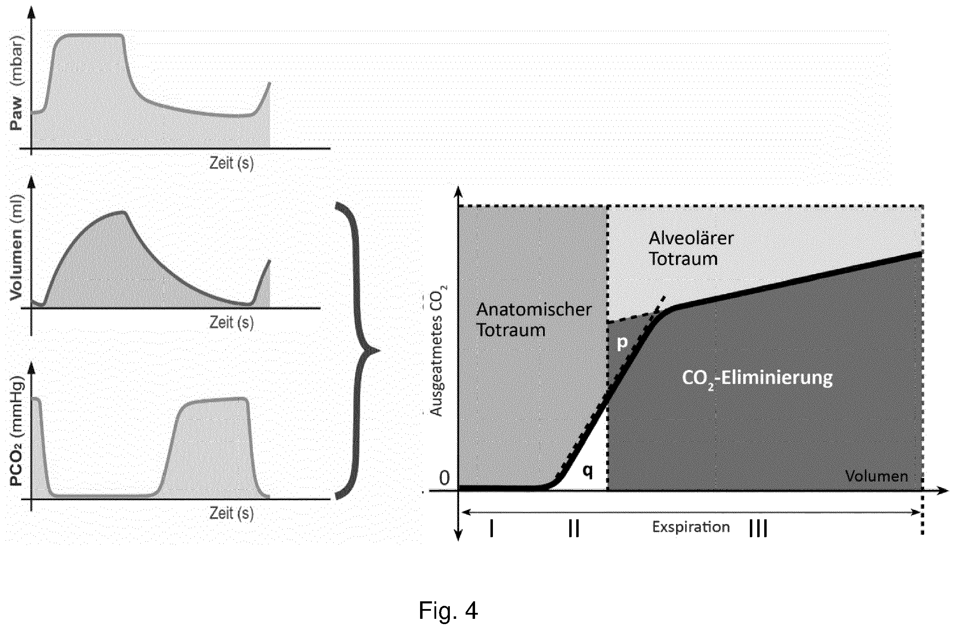

In Figur 4 sind - analog zu Fig. 3 - in der linken Hälfte die zeitbasierte Kapnographie und in der rechten Hälfte die volumenbasierte Kapnographie gegenübergestellt, wobei rechts die Anteile der Toträume eingezeichnet sind.

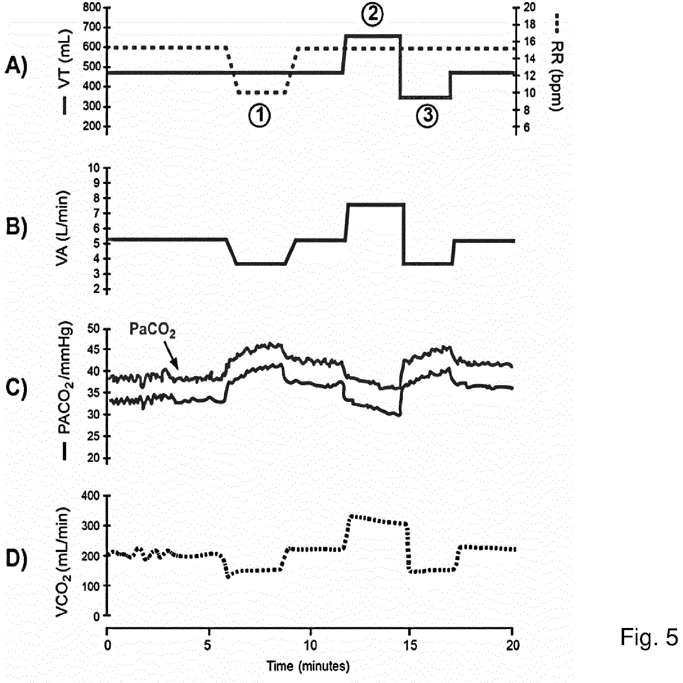

Figur 5 zeigt den Verlauf unterschiedlicher patientenspezifischer physiologischer Parameter beim Monitoring der alveolären Ventilation anhand der volumetrischen Kapnographie. Dargestellt ist die Veränderung der alveolären Ventilation (VA) bei einem hämodynamisch stabilen Patienten durch Verringerung (1) der Atemfrequenz (RR) von 15 auf 10 Atemzüge pro Minute, durch Erhöhung (2) sowie Reduktion (3) des Tidalvolumens (VT) unter kontrollierter Beatmung. Die Elimination von Kohlendioxid ($VCO_2$) und die alveolären $CO_2$-Partialdrücke ($PACO_2$) verhalten sich gegenläufig. Der arterielle $CO_2$-Partialdruck ($PaCO_2$) ist über dem alveolären $PACO_2$ (obere Linie in C) dargestellt.

Nicht alle Teile des Atemwegs - von Mund- und Nasenöffnung bis Alveolen - nehmen tatsächlich am Gasaustausch teil. Um die Effektivität der Beatmung zu beurteilen und ggf. optimieren zu können, sind Informationen über den Anteil der effektiven alveolären Ventilation sehr hilfreich. $CO_2$ ist aufgrund seiner hohen Löslichkeit und der daraus resultierenden sehr schnellen Kinetik ein idealer Indikator für die alveoläre Ventilation (VA). Daher bietet sich die Messung und grafische Darstellung des im Ausatmungsgasgemisch gemessenen $CO_2$-Partialdrucks ($PETCO_2$) in Form der Kapnographiekurve zur kontinuierlichen Überwachung der Ventilation an. Beispiele für die qualitative Beurteilung der Ventilation mittels Kapnographie sind: Diskonnektion, Apnoe, Ausschluss der Fehlintubation, Obstruktion, Ausschluss einer Patient-Ventilator-Asynchronie und vieles mehr. Weiter wird $PETCO_2$ als quantitatives Maß zur Adjustierung der Ventilation verwendet - bei stabiler Hämodynamik und konstantem Stoffwechsel ist ein hoher $PETCO_2$ Indikator einer Hypoventilation bzw. ein niedriger $PETCO_2$ Indiz für eine Hyperventilation. Beides zieht in der Praxis eine Anpassung des Atemminutenvolumens (VE) am Beatmungsgerät nach sich. Nachdem VE das Produkt aus Tidalvolumen und Atemfrequenz ist, bietet sich die Modulation dieser beiden Komponenten hierfür an. Prinzipiell werden heute niedrige Tidalvolumina (VT) angestrebt, um mittels lungenprotektiver Beatmung Volu-, Bio- und Barotraumata zu vermeiden. Was dabei jedoch besonders zu berücksichtigen ist, ist die Tatsache, dass bei jedem Atemzug neben den am Gasaustausch teilnehmenden Alveolen auch Toträume belüftet werden. Der Anteil dieser Totraumbelüftung beeinflusst die Effizienz der Beatmung maßgeblich. Für Atemminutenvolumen, alveoläre Ventilation (VA) und Totraumventilation (VD) gilt folgende einfache Formel:

$$VE = VA + VD$$

VE setzt sich also zusammen aus einem *effektiven* Teil, bei dem das Gas in Kontakt mit den Lungenkapillaren steht und damit am Gasaustausch teilnimmt (alveoläre Ventilation = VA) und einem *ineffektiven* Teil, der nicht am Gasaustausch teilnimmt (Totraum = VD). Somit ist VA und ihr Anteil an VE ein Maß für die Effizienz der Atmung und insbesondere beim respiratorisch Eingeschränkten sind VA und VD wichtige Kenngrößen zur Optimierung der Beatmungseinstellung. Demnach wird VA berechnet als:

$$VA = VE - VD$$

Abbildung 1 zeigt an einem hämodynamisch stabilen, anästhesierten Patienten die Bedeutung von VA bei der Eliminierung von $CO_2$. Änderungen der Atemfrequenz und VT-induzierte Veränderungen von VA beeinflussen die $CO_2$-Partialdrücke und die $CO_2$-Elimination ($VCO_2$) in entgegengesetztem Sinn. Die größere $VCO_2$ bei erhöhter VA reduziert die Partialdrücke auf beiden Seiten der alveolokapillären Membran, was zu einer Hypokapnie führt. Umgekehrt bringt die niedrigere VA eine geringere $CO_2$-Elimination und damit eine Hyperkapnie mit sich.

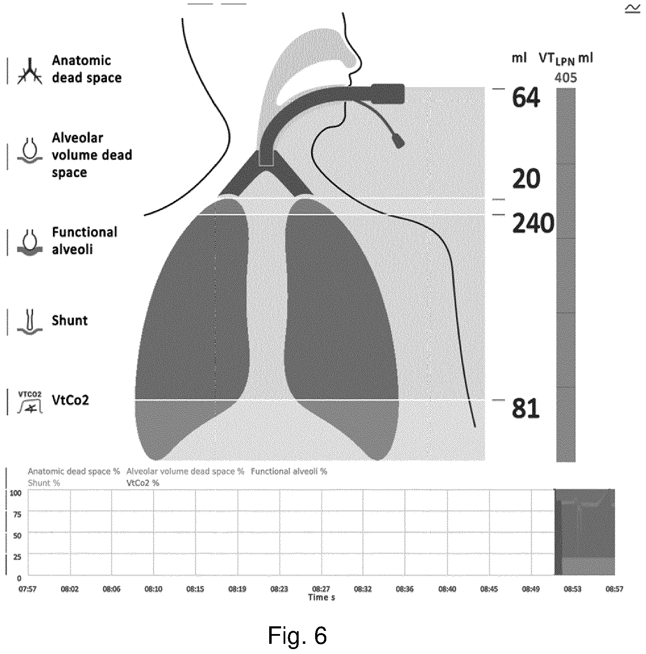

Figur 6 zeigt eine Darstellung auf dem Display des Beatmungsgeräts. Basierend auf der Erfassung wenigstens eines patientenspezifischen physiologischen Parameters wurden hier Anteile (in %) und/oder absolute Werte (in ml) für Bereiche der Lunge oder der Lungenfüllung bestimmt, die - bezogen auf zumindest den einen patientenspezifischen physiologischen Parameter - anatomischen Totraum und/oder alveoläres Totraumvolumen und/oder funktionale Alveolen und/oder Shunt und/oder $VtCO_2$ repräsentieren. Die Bereiche der Lunge sind in einer Grafik entsprechend ihren Anteilen oder absoluten Werten unterschiedlich farbig oder schraffiert, bevorzugt in einer grafischen Repräsentation der Lunge, dargestellt.

## Patentansprüche

1. Beatmungsgerät zur künstlichen Beatmung eines Patienten, umfassend:

   - eine als Pulsoxymeter und Kapnograph ausgebildete Messeinrichtung, die eingerichtet ist, um eine sich wiederholende Messung wenigstens eines patientenspezifischen physiologischen Parameters in Zeitabständen mittels Pulsoxymetrie und volumetrischer Kapnographie durchzuführen, wobei die volumetrische Kapnographie eine Menge des bei einem Atemzug eliminierten Kohlendioxids über einem ausgeatmeten Volumen des Atemzugs darstellt, wobei der wenigstens eine patientenspezifische physiologische Parameter wenigstens einem arteriellen $CO_2$-Partialdruck $PaCO_2$ und einer arteriellen Sauerstoffsättigung $SpO_2$ des Bluts des Patienten entspricht, wobei die Messeinrichtung eingerichtet ist, um den arteriellen $CO_2$-Partialdruck $PaCO_2$ über nicht invasiv gemessene Werte eines alveolären $CO_2$-Partialdrucks $PACO_2$ anzunähern, indem eine arteriell-alveoläre $CO_2$-Differenz $Pa$-$ACO_2$ bestimmt und zu den nicht invasiv gemessenen Werten hinzugefügt wird;
   - eine Steuereinrichtung, die eingerichtet ist, um wenigstens einen technischen Beatmungsparameter festzulegen, auf dessen Grundlage die Beatmung des Patienten erfolgen soll, wobei der wenigstens eine technische Beatmungsparameter wenigstens einem positiven endexspiratorischen Druck PEEP entspricht; und
   - eine Regelungseinheit, die eingerichtet ist, um mit der Messeinrichtung und der Steuereinrichtung zu kommunizieren und den wenigstens einen technischen Beatmungsparameter auf Grundlage der sich wiederholenden Messung des wenigstens einen patientenspezifischen physiologischen Parameters anzupassen.

2. Beatmungsgerät nach Anspruch 1, wobei der wenigstens eine patientenspezifische physiologische Parameter ferner wenigstens einem der folgenden Parameter entspricht: einem Atemwegstotraum $V_{Daw}$, einem alveolären Totraum $V_{Dalv}$, einem physiologischen Totraum $V_{Dphys}$, einem bei einem einzelnen Atemzug des Patienten eliminierten Volumen $VCO_2$ an $CO_2$, dem endexspiratorischen $CO_2$-Partialdruck $PETCO_2$ im ausgeatmeten Gasgemisch, dem alveolären $CO_2$-Partialdruck $PACO_2$.

3. Beatmungsgerät nach einem der vorhergehenden Ansprüche, wobei die Messeinrichtung eingerichtet ist, um die sich wiederholende Messung des wenigstens einen patientenspezifischen physiologischen Parameters nach jedem Atemzug des Patienten durchzuführen.

4. Beatmungsgerät nach einem der vorhergehenden Ansprüche, wobei die Messeinrichtung eingerichtet ist, um die sich wiederholende Messung des wenigstens einen patientenspezifischen physiologischen Parameters mittels vorzugsweise volumetrischer Oxygraphie durchzuführen, wobei der wenigstens eine patientenspezifische physiologische Parameter ferner einem alveolären $O_2$-Partialdruck $PCO_2$ und/oder einem Volumen $VO_2$ des mit einem Atemzug vom Patienten aufgenommenen Sauerstoffs entspricht.

**5.** Beatmungsgerät nach einem der vorhergehenden Ansprüche,
wobei die Messeinrichtung eingerichtet ist, um als den wenigstens einen patientenspezifischen physiologischen Parameter einen Blutvolumenfluss $PBF_{Shunt}$ des intrapulmonalen Rechts-Links-Shunts des Patienten aus einer alveolären Sauerstoffkonzentration und/oder einer Menge des mit einem Atemzug vom Patienten aufgenommenen Sauerstoffs und/oder der arteriellen Sauerstoffsättigung $SpO_2$ des Bluts des Patienten zu bestimmen.

**6.** Beatmungsgerät nach einem der vorhergehenden Ansprüche,
wobei die Steuereinrichtung eingerichtet ist, um zu Beginn der künstlichen Beatmung ein Rekrutierungsmanöver durchzuführen, wobei über eine initiale Bereitstellung eines erhöhten Beatmungsdrucks eine Lungenüberdehnung erzielt wird und dann der Beatmungsdruck reduziert und eine PEEP-Titration durchgeführt wird.

**7.** Beatmungsgerät nach einem der vorhergehenden Ansprüche, ferner umfassend:
eine Überwachungseinheit, die eingerichtet ist, um die Beatmungsdruckamplitude während der künstlichen Beatmung permanent zu überwachen, wobei ein Alarm ausgelöst wird, wenn eine voreingestellte maximale Beatmungsdruckamplitude überschritten wird.

**8.** Beatmungsgerät nach einem der vorhergehenden Ansprüche,
wobei die Steuereinrichtung eingerichtet ist, um basierend auf der Erfassung des wenigstens einen patientenspezifischen physiologischen Parameters Anteile und/oder absolute Werte für Bereiche der Lunge oder der Lungenfüllung zu bestimmen, die - bezogen auf den wenigstens einen patientenspezifischen physiologischen Parameter - anatomischen Totraum und/oder alveoläres Totraumvolumen und/oder funktionale Alveolen und/oder Shunt und/oder VtCO2 repräsentieren, wobei die Bereiche der Lunge in einer Grafik entsprechend ihren Anteilen oder absoluten Werten unterschiedlich, bevorzugt in einer grafischen Repräsentation der Lunge, dargestellt werden.

**Claims**

**1.** A breathing apparatus for artificial respiration of a patient, comprising:

• a measuring apparatus that is designed as a pulse oximeter and capnograph and is configured to perform a repeating measurement of at least one patient-specific physiological parameter at time intervals by means of pulse oximetry and volumetric capnography, wherein the volumetric capnography shows an amount of the carbon dioxide eliminated in one breath over an exhaled volume of the breath, wherein the at least one patient-specific physiological parameter corresponds at least to an arterial $CO_2$ partial pressure $PaCO_2$ and an arterial oxygen saturation $SpO_2$ of the blood of the patient, wherein the measuring apparatus is configured to approximate the arterial $CO_2$ partial pressure $PaCO_2$ through non-invasively measured values of an alveolar $CO_2$ partial pressure $PACO_2$ in that an arterial-alveolar $CO_2$ difference $Pa\text{-}ACO_2$ is determined and added to the non-invasively measured values;
• a control apparatus that is configured to establish at least one technical ventilation parameter, on the basis of which the ventilation of the patient should take place, wherein the at least one technical ventilation parameter corresponds at least to a positive end-expiratory pressure PEEP; and
• a regulating unit that is configured to communicate with the measuring apparatus and the control apparatus and to adjust the at least one technical ventilation parameter on the basis of the repeating measurement of the at least one patient-specific physiological parameter.

**2.** The breathing apparatus according to claim 1,
wherein the at least one patient-specific physiological parameter also corresponds to at least one of the following parameters: an airway dead space $V_{Daw}$, an alveolar dead space $V_{Dalv}$, a physiological dead space $V_{Dphys}$, a volume $VCO_2$ of $CO_2$ eliminated in a single breath of the patient, the end-expiratory $CO_2$ partial pressure $PETCO_2$ in the exhaled gas mixture, the alveolar $CO_2$ partial pressure $PACO_2$.

**3.** The breathing apparatus according to any one of the preceding claims,
wherein the measuring apparatus is configured to perform the repeating measurement of the at least one patient-specific physiological parameter after each breath of the patient.

**4.** The breathing apparatus according to any one of the preceding claims,
wherein the measuring apparatus is configured to perform the repeating measurement of the at least one patient-specific physiological parameter by means of preferably volumetric oxigraphy, wherein the at least one patient-specific physiological parameter also corresponds to an alveolar $O_2$ partial pressure $PCO_2$ and/or a volume $VO_2$ of the oxygen absorbed by the patient with one breath.

5. The breathing apparatus according to any one of the preceding claims,
   wherein the measuring apparatus is configured to determine a blood volume flow $PBF_{Shunt}$ of the intrapulmonary right-left shunt of the patient from an alveolar oxygen concentration and/or an amount of the oxygen absorbed by the patient with one breath and/or the arterial oxygen saturation $SpO_2$ of the blood of the patient as the at least one patient-specific physiological parameter.

6. The breathing apparatus according to any one of the preceding claims,
   wherein the control apparatus is configured to perform a recruiting maneuver at the beginning of the artificial respiration, wherein an overexpansion of the lungs is achieved by initially providing an increased ventilation pressure, and then the ventilation pressure is reduced and a PEEP titration is performed.

7. The breathing apparatus according to any one of the preceding claims, further comprising:
   a monitoring unit that is configured to continuously monitor the ventilation pressure amplitude during artificial respiration, wherein an alarm is triggered if a preset maximum ventilation pressure amplitude is exceeded.

8. The breathing apparatus according to any one of the preceding claims,
   wherein the control apparatus is configured to determine, based on the detection of the at least one patient-specific physiological parameter, proportions and/or absolute values for regions of the lung or the lung filling, which - in relation to the at least one patient-specific physiological parameter - represent anatomic dead space and/or alveolar dead space volume and/or functional alveoli and/or shunt and/or $VtCO_2$, wherein the regions of the lung are shown differently in a graphic according to their proportions or absolute values, preferably in a graphical representation of the lung.

**Revendications**

1. Appareil respiratoire destiné à la ventilation artificielle d'un patient, comportant :

   - un dispositif de mesure conçu comme un oxymètre de pouls et capnographe, lequel est configuré pour effectuer une mesure répétitive d'au moins un paramètre physiologique spécifique au patient à des intervalles de temps par oxymétrie de pouls et capnographie volumétrique, dans lequel la capnographie volumétrique indique une quantité du dioxyde de carbone éliminé dans une respiration par un volume expiré de la respiration, dans lequel l'au moins un paramètre physiologique spécifique au patient correspond à au moins une pression partielle de $CO_2$ artérielle $PaCO_2$ et à une saturation en oxygène artérielle $spO_2$ du sang du patient, dans lequel le dispositif de mesure est configuré pour rapprocher la pression partielle de $CO_2$ artérielle $PaCO_2$ d'une pression partielle de $CO_2$ alvéolaire $PACO_2$ par des valeurs mesurées de façon non invasives, en déterminant une différence de $CO_2$ artérielle-alvéolaire $Pa\text{-}ACO_2$ et en l'ajoutant aux valeurs mesurées de façon non invasive ;
   - un dispositif de commande configuré pour définir au moins un paramètre de ventilation technique, sur la base duquel la ventilation du patient doit s'effectuer, dans lequel l'au moins un paramètre de ventilation technique correspond à au moins une pression de fin d'expiration positive PEEP ; et
   - une unité de réglage configurée pour communiquer avec le dispositif de mesure et le dispositif de commande et pour adapter l'au moins un paramètre de ventilation technique sur la base de la mesure répétitive de l'au moins un paramètre physiologique spécifique au patient.

2. Appareil respiratoire selon la revendication 1,
   dans lequel l'au moins un paramètre physiologique spécifique au patient correspond en outre à l'un au moins des paramètres suivants : un espace mort de voie respiratoire $V_{Daw}$, un espace mort alvéolaire $V_{Dalv}$, un espace mort physiologique $V_{Dphys}$, un volume de $VCO_2$ et de $CO_2$ éliminé lors d'une seule respiration du patient, la pression partielle de $CO_2$ de fin d'expiration $PETCO_2$ dans le mélange gazeux expiré, la pression partielle de $CO_2$ alvéolaire $PACO_2$.

3. Appareil respiratoire selon l'une des revendications précédentes,
   dans lequel le dispositif de mesure est configuré pour effectuer la mesure répétitive de l'au moins un paramètre physiologique spécifique au patient après chaque respiration du patient.

**4.** Appareil respiratoire selon l'une des revendications précédentes,
dans lequel le dispositif de mesure est configuré pour effectuer la mesure répétitive de l'au moins un paramètre physiologique spécifique au patient de préférence par oxygraphie volumétrique, dans lequel l'au moins un paramètre physiologique spécifique au patient correspond en outre à une pression partielle d'$O_2$ alvéolaire $PCO_2$ et/ou à un volume $VO_2$ de l'oxygène absorbé par le patient avec une respiration.

**5.** Appareil respiratoire selon l'une des revendications précédentes,
dans lequel le dispositif de mesure est configuré pour déterminer, en tant qu'au moins un paramètre physiologique spécifique au patient, un débit volumique sanguin $PBF_{Shunt}$ du shunt droite-gauche intrapulmonaire du patient à partir d'une concentration en oxygène alvéolaire et/ou d'une quantité de l'oxygène absorbé par le patient avec une respiration et/ou la saturation en oxygène artérielle $SpO_2$ du sang du patient.

**6.** Appareil respiratoire selon l'une des revendications précédentes,
dans lequel le dispositif de commande est configuré pour effectuer une manoeuvre de recrutement au début de la ventilation artificielle, dans lequel, par une mise à disposition initiale d'une pression de ventilation augmentée, une sur-dilatation pulmonaire est atteinte et la pression de ventilation est ensuite réduite et un titrage PEEP est effectué.

**7.** Appareil respiratoire selon l'une des revendications précédentes, comportant en outre :
une unité de surveillance configurée pour surveiller en permanence l'amplitude de pression de ventilation pendant la ventilation artificielle, dans lequel une alarme est déclenchée lorsqu'une amplitude de pression de ventilation maximale préréglée est dépassée.

**8.** Appareil respiratoire selon l'une des revendications précédentes,
dans lequel le dispositif de commande est configuré pour déterminer, sur la base de la détection de l'au moins un paramètre physiologique spécifique au patient, des parts et/ou des valeurs absolues pour des zones pulmonaires ou pour le remplissage pulmonaire, lesquelles - par rapport à l'au moins un paramètre physiologique spécifique au patient - représentent un espace mort anatomique et/ou un volume d'espace mort alvéolaire et/ou des alvéoles fonctionnelles et/ou un shunt et/ou un $VtCO_2$, dans lequel les zones pulmonaires sont indiquées de différentes façons dans un graphique en fonction de leurs parts ou valeurs absolues, de préférence dans une représentation graphique des poumons.

Fig. 1

Fig. 2

Fig. 3

Fig. 4

18

Fig. 5

ml VT$_{LPN}$ ml
405

64

20

240

81

Anatomic dead space

Alveolar volume dead space

Functional alveoli

Shunt

VtCo2 VtCo2

Anatomic dead space %  Alveolar volume dead space %  Functional alveoli %
Shunt %                 VtCo2 %

100  75  50  25  0

07:57  08:02  08:06  08:10  08:15  08:19  08:23  08:27  08:32  08:36  08:40  08:45  08:49  08:53  08:57

Time s

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005092415 A1
- US 20120145152 A1
- US 20080202525 A1
- WO 2013163735 A1
- US 20050109340 A1
- US 2007068528 A1
- US 2007163584 A1

**In der Beschreibung aufgeführte Nicht-Patentliteratur**

- **ENGHOFF H.** Volum inefficax. Bemerkungen zur Frage des schädlichen Raumes. *Upsala Läk Fören Förch,* 1938, vol. 44, 191-218